# EUROPEAN PATENT SPECIFICATION

(11) **EP 1 385 560 B1**
(45) Date of publication and mention of the grant of the patent: **16.05.2018**
(21) Application number: 02707800.5
(22) Date of filing: 15.02.2002
(51) Int. Cl.: A61M 5/32, A61M 5/46, A61D 7/00

(54) **PREFILLABLE INTRADERMAL DELIVERY DEVICE WITH HIDDEN NEEDLE AND PASSIVE SHIELDING**
VORFÜLLBARE INTRADERMALE ZUFUHRVORRICHTUNG MIT VERSTECKTER NADEL UND PASSIVEM SCHUTZ
DISPOSITIF D'ADMINISTRATION INTRADERMIQUE PREREMPLISSABLE A AIGUILLE CACHEE ET PROTECTION PASSIVE

(30) Priority: 13.04.2001 US 834438; 13.04.2001 US 834669; 25.07.2001 WO PCT/US01/23367
(43) Date of publication of application: 04.02.2004
(73) Proprietor: Becton, Dickinson and Company, Franklin Lakes, NJ 07417-1880 (US)
(72) Inventor: ALCHAS, Paul, G., Wayne, NJ 07470 (US); GUILLERMO, Carlos, E., Clinton, CT 06413 (US); DESALVO, David, Butler, NJ 07405 (US); GIAMBATTISTA, Lucio, East Hanover, NJ 07936-1809 (US)
(74) Representative: dompatent von Kreisler Selting Werner - Partnerschaft von Patent- und Rechtsanwälten mbB
(86) International application number: PCT/US2002/004703
(87) International publication number: WO 2002/083205

(56) References cited:
- EP-A2- 1 066 848
- WO-A-99/22790
- DE-C- 958 766
- GB-A- 2 079 607
- US-A- 4 373 526
- US-A- 5 190 521
- US-A- 5 417 660
- US-A- 5 417 662
- US-A- 5 634 906
- US-A- 5 980 495
- US-A- 6 056 716

## Description

### FIELD OF THE INVENTION

The present invention relates generally to an intradermal injection assembly having a limiter positioned to limit the penetration of a forward tip of a needle cannula into the dermis of an animal. More particularly, the present invention relates to an intradermal delivery assembly having an integrated, passive shielding device to shield the forward tip of the needle cannula prior to and subsequent to administering the intradermal injection.

### BACKGROUND OF THE INVENTION

Intradermal injections are used for delivering a variety of substances. Many of these substances have proven to be more effectively absorbed into or react with the immune response system of the body when injected intradermally. An intradermal injection is made by delivering the substance into the epidermis and upper layer of the dermis. Below the dermis layer is subcutaneous tissue (also sometimes referred to as the hypodermis layer) and muscle tissue, in that order. There is considerable variation in the skin thickness both between individuals and within the same individual at different sites of the body. Generally, the outer skin layer epidermis has a thickness of between 50 to 200 microns, and the dermis, the inner and thicker layer of the skin, has a thickness between 1.5 and 3.5 mm. Therefore, a needle cannula that penetrates the skin deeper than about 3.0 mm has a potential of passing through the dermis layer of the skin making the injection into the subcutaneous region, which may result in an insufficient immune response, especially where the substance to be delivered intradermally has not been indicated for subcutaneous injection. Also, the needle cannula may penetrate the skin at too shallow a depth to deliver the substance and result in what is commonly known in the art as "wet injection" due to reflux of the substance from the injection site.

Due to the inherent limitations of the standard needle assembly, the standard procedure for making an intradermal injection is known to be difficult to perform, and therefore dependent upon experience and technique. This procedure is recommended to be performed by stretching the skin, orienting the needle bevel to face upwardly, and inserting a 26 gauge short bevel needle cannula to deliver a volume of .5 ml or less of the substance into the skin of the animal with the needle cannula being inserted into the skin at an angle varying from around 10 to 15° to form a blister or wheel in which the substance is deposited or otherwise contained.

The technique utilized to perform the standard intradermal injection is difficult and requires the attention of a trained nurse or medical doctor. Inserting the needle to a depth greater than about 3.0 mm typically results in a failed intradermal injection because the substance being expelled through the cannula will be injected into the subcutaneous tissue of the animal. As disclosed in pending United States Patent Application Nos. 09/834,438 and 09/417,671, an intradermal needle assembly has been developed for use with a prefillable container having a reservoir capable of storing a substance for injection into the skin of an animal. A hub portion, which is attachable to the prefillable container storing the substance, supports a needle cannula having a forward tip extending away from the hub portion. A limiter portion surrounds the needle cannula and extends away from the hub portion towards the forward tip of the needle cannula. The limiter includes a generally flat skin engaging surface extending in a plane generally perpendicular to an axis of the needle cannula and is adapted to be received against the skin of the animal to administer the intradermal injection of the substance. The needle forward tip extends beyond the skin engaging surface a distance of approximately 0.5 to 3.0 mm. Therefore, the limiter portion limits penetration of the needle into the dermis layer of the skin of the animal so that the vaccine is injected into the dermis layer of the animal.

The devices that have been adapted to administer an intradermal injection, and other devices utilizing needle cannulas with sharp tips to inject substances into the skin of the animal have typically included a fixed design where each of the components of the assembly are stationary relative to the other components. Therefore, the forward tip of the needle cannula remains exposed even after administration of the intradermal injection. With the advent of viral diseases transmitted through the contact of body fluid, it has become desirable to develop needle shielding devices that would prevent unintended access to the needle cannula subsequent to administering an injection. While these types of devices have been introduced to the marketplace for standard injection assemblies, they have not been developed in combination with an injection device adapted to administer an intradermal injection through the use of a limiter as described above.

In addition, it has become increasingly desirable to hide the needle prior to use to address any potential concerns by patients of seeing an exposed needle prior to use.

Therefore, it would be desirable to introduce an intradermal delivery assembly utilizing a limiter, as set forth above, to limit the penetration of a needle cannula into the dermis of an animal in combination with a shielding component to prevent unintended contact with the needle subsequent to administration of the intradermal injection, and to hide the needle prior to use.

An automatic syringe is disclosed in WO 99/22790. This needle assembly comprises a barrel containing a reservoir communicating with a delivery needle. The needle is covered before use by a removable sheath, and after the sheath is removed, the needle is concealed by a displaceable sleeve. In use, the sleeve is pressed against the skin by applying pressure while holding the barrel. The sleeve is thereby retracted into the barrel allowing the needle to penetrate the skin. The movement of the sleeve activates a gas generator which expels the liquid from the needle.

In GB 2 079 607, there is described a holder for a hypodermic syringe. The holder comprises a sleeve having a prefillable container fixedly disposed therein, and a limiter slidable protruding from the sleeve. The limiter is freely movable between a first position and a second position, and there is no catch to prevent said limiter from moving from the first position to the second position subsequent to administering an intradermal injection. The proximal end of the prefillable container is fixedly connected with the proximal end of the sleeve, and a spring urging the limiter in distal direction with respect to the sleeve is positioned within the limiter.

US 5,634,906 A discloses a needle hiding shield to be coupled to a dose metering syringe. In one embodiment, a medication cartridge housing is surrounded by an outer spring sleeve. The distal end of the medication cartridge housing has a circumferential annular gap engaging with a peripheral lip of a retaining collar connected with the sleeve. A spring housed within the sleeve is supported by the retaining collar and urges a needle guard in distal A combination of an intradermal needle assembly and a prefillable container having a reservoir according to the pre-amble of claim 1 is disclosed in in US 5,190,521.

### SUMMARY OF THE INVENTION AND ADVANTAGES

It is an object of the invention to provide a combination of a prefillable container having a reservoir and an intradermal needle assembly that limits the penetration of a needle cannula into the dermis of an animal in combination with a shielding component.

The invention is defined by claim 1. Preferred embodiments are defined in dependent claims 2-5.

The intradermal needle assembly of the present invention is combined with a prefillable container that includes a reservoir capable of storing a substance for injection into the skin of an animal. A hub portion is attachable to the prefillable container storing the substance and supports a needle cannula. The needle cannula includes a forward tip extending away from the hub portion. A limiter surrounds the needle cannula and extends away from the hub portion towards the forward tip of the needle cannula. The limiter includes a generally flat skin engaging surface extending in a plane generally perpendicular to an axis of the needle cannula and is adapted to be received against the skin of the animal to administer an intradermal injection of the substance.

The limiter is moveable between at least a first position where the skin engaging surface shields the forward tip of the needle cannula, and a second position where the forward tip extends beyond the skin engaging surface a distance sufficient to administer the substance into the dermis layer of the animal upon depression of the skin engaging surface against the skin of the animal. The limiter is biased toward the first position by a spring means that is compressible upon depressing the skin engaging surface against the skin of the animal. Therefore, after administering the intradermal injection and withdrawing the skin engaging surface from the skin of the animal, the limiter returns and locks to the first position to shield the forward tip of the needle cannula.

The embodiments of the subject invention set forth above provide the benefit of administering an intradermal injection utilizing a depth limiting device that limits the penetration of the forward tip of the needle cannula into the dermis layer of the animal simplifying the method of administering the intradermal injection. Further, a passive shielding device is provided which shields the forward tip of the needle cannula both prior to and subsequent to administering the intradermal injection. Thus, the intradermal needle assembly prevents exposure to the forward tip of the needle cannula prior to administering the injection maintaining sterilization, and subsequent to administering the injection reducing the potential for a biohazard resulting from contact with the needle cannula.

### BRIEF DESCRIPTION OF THE DRAWINGS

Other advantages of the present invention will be readily appreciated as the same becomes better understood by reference to the following detailed description when considered in connection with the accompanying drawings wherein:
Figure 1 is cross-sectional view of a first embodiment of the present invention showing a needle limiter capable of shielding a needle cannula while located in a first position;
Figure 2 is cross-sectional view of the first embodiment of the present invention showing a needle limiter limiting the injection depth of the needle cannula while located in a second position;
Figure 3 is cross-sectional view of the first embodiment of the present invention showing a needle limiter shielding a needle cannula while locked in the first position subsequent to administering the intradermal injection;
Figure 4 is an exploded view of the first embodiment of the present invention;
Figures 5 and 6 show an alternate embodiment of the present invention in the first and second positions;
Figure 7 shows the alternate embodiment locked in the first position subsequent to administering the intradermal injection;
Figure 8 shows a further embodimen not belonging to the present invention with a sleeve capable of shielding the needle cannula while in a first position;
Figure 9 shows the further embodiment with the sleeve located in a retracted position while administering the intradermal injection;
Figure 10 shows the further embodiment locked in the first position subsequent to administering the intradermal injection;
Figures 11 and 12 show a still further embodiment not belonging to the present invention having a limiter slidably attached to a hub and being capable of shielding the needle cannula prior to and subsequent to administering the intradermal injection; and
Figure 13 shows the still further embodiment locked in the first position subsequent to administering the intradermal injection.

### DETAILED DESCRIPTION OF THE PREFERRED EMBODIMENT

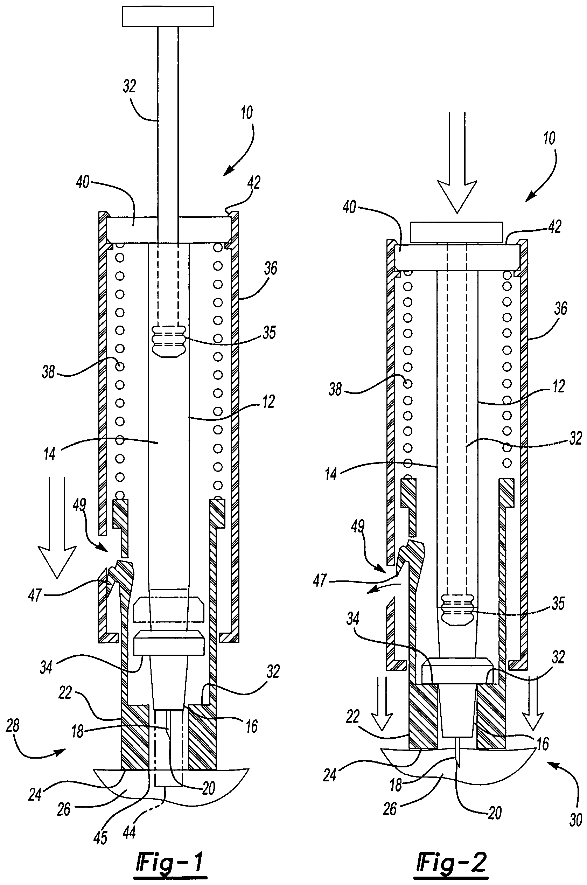

An intradermal needle assembly of the subject invention is generally shown at 10 of Figures 1, 2 and 3. The assembly includes a prefillable container 12 having a reservoir 14 capable of storing a substance for injection into the skin of an animal. A hub portion 16 is attachable to the prefillable container 12 and supports a needle cannula 18 having a forward tip 20 extending away from the hub portion 16.

A limiter 22 surrounds the needle cannula and extends away from the hub portion 16 toward the forward tip 20 of the needle cannula 18. The limiter 22 includes a generally flat skin engaging surface 24 extending in a plane generally perpendicular to an axis of the needle cannula 18. The skin engaging surface 24 is adapted to be received against the skin 26 of an animal to administer an intradermal injection of the substance.

The limiter 22 is moveable between a first position 28 as shown in Figure 1 and a second position 30 as shown in Figure 2. When the limiter is located in the first position 28, the skin engaging surface 24 shields the forward tip 20 of the needle cannula 18. When the limiter 22 is located in the second position 30, the forward tip 20 of the needle cannula 18 extends beyond the skin engaging surface 24 a distance sufficient to administer the substance disposed in the reservoir 14 into the dermis layer 26 of the animal. The assembly 10 includes a plunger 32 having a stopper 35 slideably disposed within the reservoir 14 of the prefillable container 12. As known to those of skill in the art, by depressing the plunger 32, the stopper 35 slides inwardly of the reservoir 14 causing the substance disposed in the reservoir 14 to be expelled from the prefillable container 12 through the needle cannula 18 into the skin 26 of the animal.

When the limiter 22 is disposed in the second position 30, the forward tip 20 of the needle cannula 18 extends beyond the skin engaging surface 24 a distance of approximately 0.5 to 3.0 mm. Therefore, the penetration of the needle cannula 18 into the dermis layer of the skin 26 is limited so that the substance is injected into the dermis layer 26 of the animal. The limiter 22 includes at least one stop 32 that is engageable with a counter stop 34 disposed upon the hub portion 16 to terminate movement of the limiter 22 rearwardly of the forward tip 20, thereby allowing only 0.5 to 3.0 mm of the needle cannula 18 to be exposed.

The limiter 22 is slideably disposed within a sleeve 36. Accordingly, the limiter 22 is concentrically aligned between the sleeve 36 and the prefillable container 12. The limiter 22 is biased outwardly of the sleeve 36 by a spring means 38. The spring means is represented in the figures as a coil spring, however, it should be understood by those of skill in the art that any resilient member may be used to bias the limiter 22 outwardly of the sleeve 36.

The spring means 38 is compressed between the limiter 22 and a rim 40 of the prefillable container 12 disposed upon an end of the prefillable container 12 opposite the limiter 22. The rim 40 of the prefillable container 12 is fixedly attached to the sleeve 36 by a plurality of snaps 42. A plug 44 is engageable with the needle cannula 18 to seal the prefillable container 12 at the needle cannula 18. The plug 44 is inserted through a central aperture 45 in the skin engaging surface 24 that is axially aligned with the needle cannula 18. The plug 44 is removed prior to administering the intradermal injection.

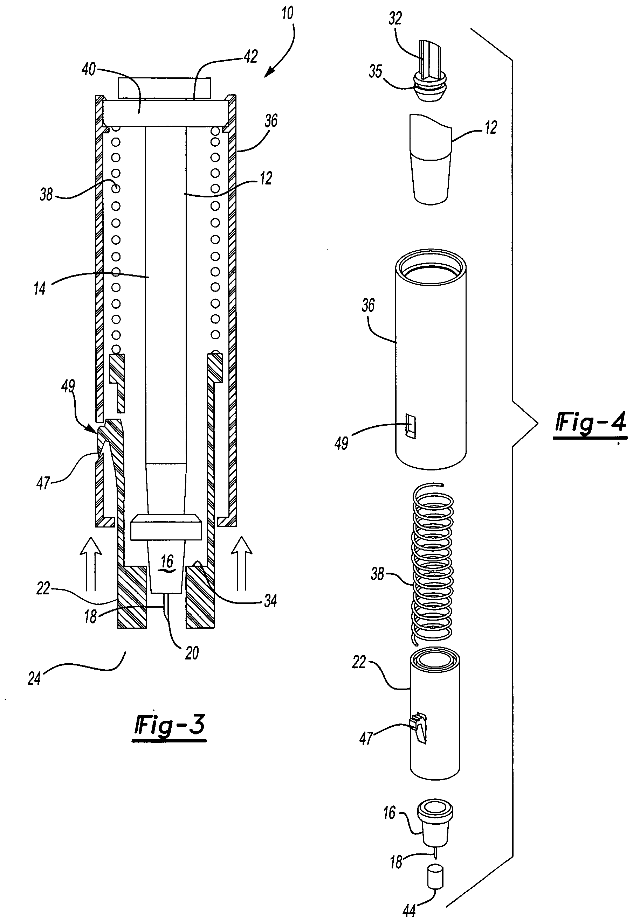

A least one catch 47 is disposed upon a side of the limiter 22 and is engageable with a slot 49 disposed in a side of the sleeve 36. The catch 47 is biased to engage the slot 49 when the limiter 22 returns to the first position 28 subsequent to administering the injection to prevent the forward tip 20 from being re-exposed. This is best represented in Figure 3.

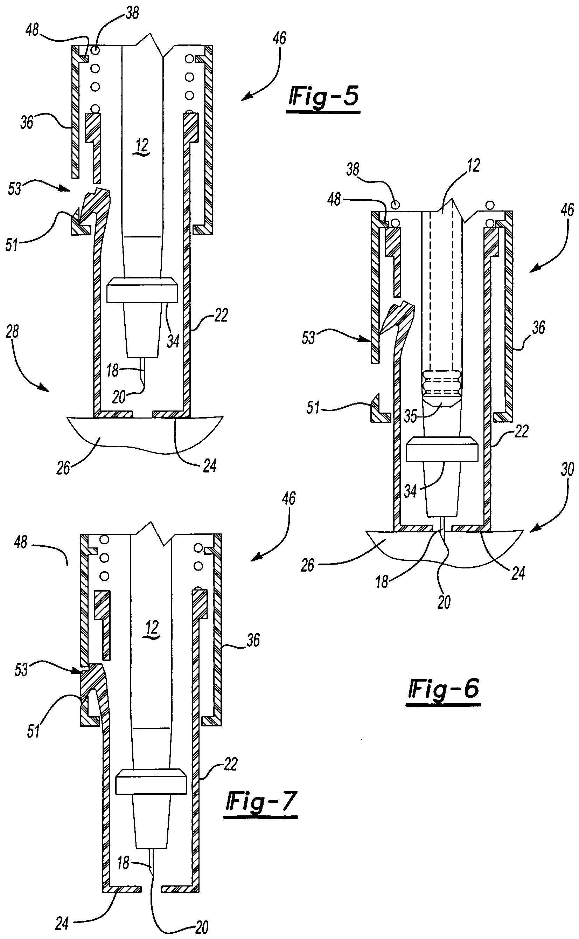

Figures 5, 6 and 7 show an alternate embodiment of the subject assembly generally at 46. At least one sleeve stop 48 is positioned rearwardly of the limiter 22 inside the sleeve 36. The sleeve stop 48 is spaced from the limiter 22 when the limiter is located in the first position 28. Movement of the limiter by depressing the skin engaging surface 24 against the skin 26 of the animal is terminated when the limiter 22 abuts the sleeve stop 48 locating the limiter 22 in the second position 30. As set forth above, when the limiter 22 is located in the second position 30, the forward tip 20 of the needle cannula 18 extends beyond the skin engaging surface 24 a distance between 0.5 and 3.0 mm. Upon withdrawing the alternate embodiment 46 from the skin 26 of the animal, the limiter 22 returns to the first position 28 shielding the needle cannula 18.

A least one catch 51 is disposed upon a side of the limiter 22 and is engageable with a slot 53 disposed in a side of the sleeve 36. The catch 51 is biased to engage the slot 53 when the limiter 22 returns to the first position 28 subsequent to administering the injection to prevent the forward tip 20 from being re-exposed. This is best represented in Figure 7.

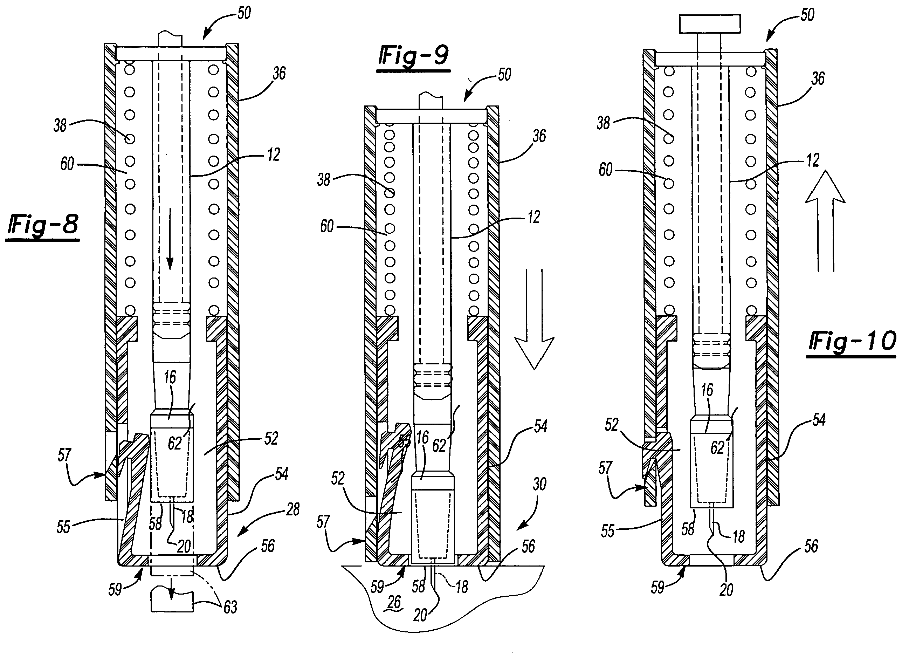

Figures 8 and 9 show an example outside of the scope of the present invention generally at 50. A limiter portion 52 is affixed to the hub portion 16 in a stationary manner. A shield 54 circumscribes the limiter 52 and is concentrically aligned between the sleeve 36 and the prefillable container 12.

The shield 54 is slideable between the first position 28 and the second position 30. The spring means 38 biases the shield 54 towards the first position 28. The shield 54 shields the forward tip 20 of the needle cannula 18 when in the first position 28. The shield includes a skin engaging surface 56 that aligns with a generally flat skin engaging surface 58 of the stationary limiter portion 52 when the shield 54 is located in the second position 30. Upon depressing the limiter skin engaging surface 58 against the skin 26 of an animal, the shield 54 moves inwardly of the sleeve 36 exposing 0.5 to 3.0 mm of the needle cannula enabling the intradermal injection to be made into the skin 26 of the animal. Upon withdrawing the assembly 50 from the skin 26 of the animal, the shield 54 moves outwardly of the sleeve 36 returning to the first position 28. An abutting member 62 abuts the hub portion 16 preventing the shield 54 from moving beyond the first position 28 or being ejected from the assembly 50 by the spring means 38.

A least one catch 55 is disposed upon a side of the limiter 22 and is engageable with a slot 57 disposed in a side of the sleeve 36. The catch 55 is biased to engage the slot 57 when the limiter 22 returns to the first position 28 subsequent to administering the injection to prevent the forward tip 20 from being re-exposed. This is best represented in Figure 10.

A plug 63 is engageable with the needle cannula 18 to seal the prefillable container 12 at the needle cannula 18. The plug 63 is inserted through a central aperture 59 in the skin engaging surface 56 that is axially aligned with the needle cannula 18. The plug 63 is removed prior to administering the intradermal injection.

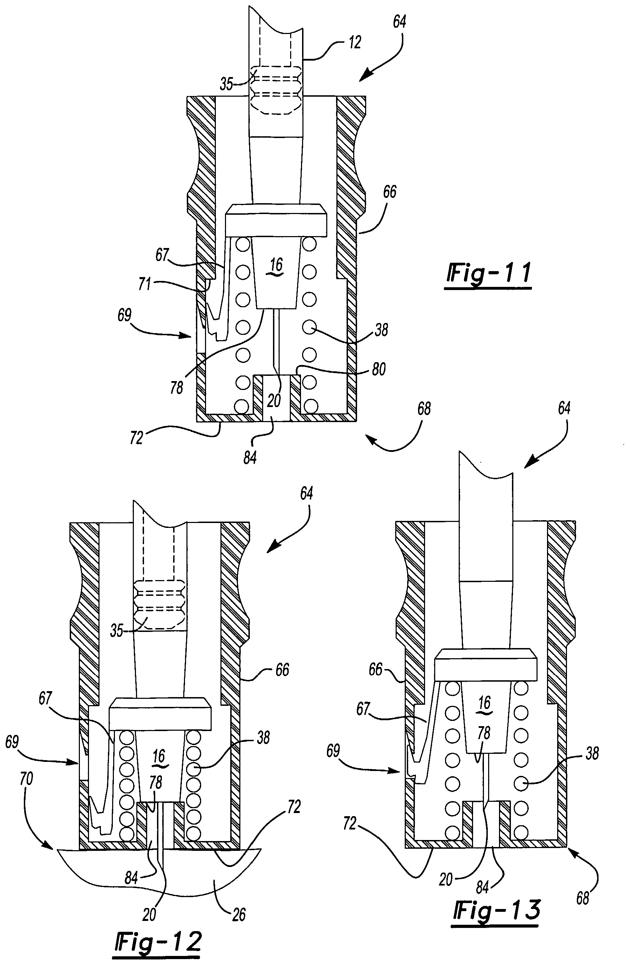

An additional example outside of the scope of the present invention is generally shown at 64 in Figures 11 through 13. An alternate limiter 66 circumscribes the hub portion 16 and slideably engages the prefillable container 12.

The alternate limiter 66 is moveable between a first position 68 and a second position 70 relative to the hub portion 16. A skin engaging surface 72 as disposed upon the limiter 66 is engageable with the skin of the animal to administer the intradermal injection. The spring means 38 is compressed between the hub portion 16 and a rear side of the skin engaging surface 72. The spring means 38 biases the alternate limiter 66 to the first position 68. Upon depression of the skin engaging surface 72 against the skin 26 of the animal, the spring means 38 is compressed allowing the alternate limiter 66 to move from the first position 68 to the second position 70.

The hub portion 16 includes at least one catch 67 extending towards the forward tip 20 of the needle cannula 16. The catch 67 engages a slot 69 disposed in a wall of the limiter 66 to prevent the limiter 66 from being moved to second position 70 from the first position subsequent to administering the injection. A plurality of catches 67 may also be used to improve the stability of the limiter 66. A stop 71 is disposed upon an inner wall of the limiter and is engageable with the catch 67 to prevent the limiter 66 from being ejected from the assembly 64 by the spring means 38.

The forward tip 20 of the needle cannula 18 extends beyond the skin engaging surface 72 a distance of 0.5 to 3.0 mm when the alternate limiter 66 is disposed in the second position 70.

As shown in Figures 10 and 11, the alternate limiter 66 includes a stop 74 disposed upon an inner surface and is engageable with a rear member 76 of the hub portion 16. The stop 74 prevents the alternate limiter 66 from moving beyond the second position 70. Alternatively, as shown in Figures 12 and 13, a leading edge 78 of the hub portion 16 engages a flange 80 directed rearwardly from the skin engaging surface 72 to prevent the limiter portion 66 from moving beyond the second position. A plug 82 is engageable with the needle cannula 18 to seal the prefillable container 12 at the needle cannula. The plug 82 is inserted through a central aperture 84 in the skin engaging surface 72 that is axially aligned with the needle cannula 18. The plug 82 is removed prior to administering the intradermal injection.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, wherein reference numerals are merely for convenience and are not to be in any way limiting, the invention may be practiced otherwise than as specifically described.

## Claims

1. A combination of an intradermal needle assembly and a prefillable container (12) having a reservoir (14) capable of storing a substance for injection into the skin of an animal comprising:
a hub portion (16) being attachable to the prefillable container storing the substance;
a needle cannula (18) supported by said hub portion and having a forward tip (20) extending away from said hub portion;
a limiter (22) surrounding said needle cannula and extending away from said hub portion toward said forward tip of said needle cannula,
said limiter (22) including a generally flat skin engaging surface (24) extending in a plane generally perpendicular to an axis of said needle cannula (18) for limiting the penetration of the forward tip of the needle cannula into the dermis layer of the animal and a central aperture (45) in the skin engaging surface (24) that is axially aligned with the needle cannula (18), said limiter adapted to be received against the skin of the animal to administer an intradermal injection of the substance; **characterised by**;
a sleeve (36) having a slot (49, 53) therein and having said prefillable
container fixedly disposed therein with said limiter (22) slideably protruding therefrom,
said limiter (22) being movable between at least a first position (28),
wherein said skin engaging surface (24) shields said forward tip of said needle cannula, and a second position (30), wherein said forward tip extends beyond said skin engaging surface a distance sufficient to administer the substance into the dermis layer of the animal upon depression of said skin engaging surface against the skin of the animal,
wherein said limiter (22) is biased towards said first position (28) with a spring means (38), and
at least one catch (47, 51) engageable with the slot (49, 53) in said sleeve (36) to prevent said limiter (22) from moving from said first position to said second position subsequent to administering the intradermal injection,
the container (12) having a rim (40) fixedly attached to the sleeve (36), said rim being disposed on an end opposite said hub (16) and said spring means (38) being compressed between said rim (40) and said limiter (22), and
a plunger (32) having a stopper (35) within the container (12) and extending out of the sleeve (36).

2. An assembly as set forth in claim 1 wherein said forward tip (20) of said needle cannula (18) extends beyond said skin engaging surface (24) a distance approximately 0,5 mm to 3,0 mm when said limiter (22) is located in said second position (30) thereby limiting penetration of said needle into the dermis layer of skin of the animal so that the substance is injected into the dermis layer of the animal.

3. An assembly as set forth in claim 1 wherein said sleeve (36) includes at least one stop positioned to prevent said limiter (22) from sliding beyond said second position (30).

4. An assembly as set forth in claim 1 wherein said rim (40) is attached to the sleeve (36) by a plurality of snaps.

5. An assembly as set forth in claim 1 further including a plug (44) comprising a resilient material applied over said forward tip (20) of said needle cannula thereby sealing said prefillable container (12) at said needle cannula (19).

## Patentansprüche

1. Kombination aus einer intradermalen Nadelanordnung und einem vorbefüllbaren Behälter (12) mit einem Reservoir (14), das in der Lage ist, eine in die Haut eines Tieres zu injizierende Substanz aufzunehmen, mit:
einem Ansatzbereich (16), der an dem die Substanz enthaltenden vorbefüllbaren Behälter anbringbar ist;
einer Nadelkanüle (18), die von dem Ansatzbereich gestützt ist und eine vordere Spitze (20) aufweist, die sich von dem Ansatzbereich weg erstreckt;
einem Begrenzer (22), der die Nadelkanüle umgibt und sich von dem Ansatzbereich in Richtung der vorderen Spitze der Nadelkanüle erstreckt,
wobei der Begrenzer (22) eine im Wesentlichen flache Hautanlagefläche (24) aufweist, die sich in einer zu einer Achse der Nadelkanüle (18) im Wesentlichen senkrechten Ebene erstreckt, um das Eindringen der vorderen Spitze der Nadelkanüle in die Dermisschicht des Tieres zu begrenzen, und eine mittige Öffnung (45) in der Hautanlagefläche (24) aufweist, die axial mit der Nadelkanüle (18) ausgerichtet ist, wobei der Begrenzer geeignet ist, auf der Haut des Tieres aufgenommen zu werden, um eine intradermale Injektion der Substanz zu verabreichen,
**gekennzeichnet durch**:
eine Hülse (36), der einen darin ausgebildeten Schlitz (49, 53) aufweist und in welchem der vorbefüllbare Behälter fest angeordnet ist, wobei der Begrenzer (22) gleitend verschiebbar aus dieser ragt,
wobei der Begrenzer (22) zwischen mindestens einer ersten Position (28), in welcher die Hautanlagefläche (24) die vordere Spitze der Nadelkanüle abschirmt, und einer zweiten Position (30) bewegbar ist, in welcher sich die vordere Spitze über eine Strecke über die Hautanlagefläche hinaus erstreckt, die ausreicht, um beim Niederdrücken der Hautanlagefläche gegen die Haut des Tieres die Substanz in die Dermisschicht des Tieres auszugeben,
wobei der Begrenzer (22) durch eine Federeinrichtung (38) in Richtung der ersten Position (28) vorgespannt ist, und
mindestens eine Raste (47, 51), die in Eingriff mit dem Schlitz (49, 53) in der Hülse (36) bringbar ist, um den Begrenzer (22) nach der Ausgabe der intradermalen Injektion an einer Bewegung von der ersten Position in die zweite Position zu hindern,
wobei der Behälter (12) einen Rand (40) aufweist, der fest an der Hülse (36) angebracht ist, wobei der Rand an einem dem Ansatz (16) entgegengesetzte Ende angeordnet ist, und die Federeinrichtung (38) zwischen dem Rand (40) und dem Begrenzer (22) zusammengedrückt ist, und
einen Kolben (32), der einen Stopfen (35) in dem Behälter (12) aufweist und sich aus der Hülse (36) heraus erstreckt.

2. Anordnung nach Anspruch 1, bei welcher die vordere Spitze (20) der Nadelkanüle (18) sich über eine Strecke von ungefähr 0,5 mm bis 3,0 mm über die Hautanlagefläche (24) hinaus erstreckt, wenn sich der Begrenzer (22) in der zweiten Position (30) befindet, wodurch er das Eindringen der Nadel in die Dermisschicht der Haut des Tieres begrenzt, so dass die Substanz in die Dermisschicht des Tieres injiziert wird.

3. Anordnung nach Anspruch 1, bei welcher die Hülse (36) mindestens einen Anschlag aufweist, der derart positioniert ist, dass er den Begrenzer (22) daran hindert, über die zweite Position (30) hinaus zu gleiten.

4. Anordnung nach Anspruch 1, bei welcher der Rand (40) an der Hülse (36) durch mehrere Schnappeinrichtungen befestigt ist.

5. Anordnung nach Anspruch 1, ferner mit einem Pfropfen (44) mit einem elastischen Material, das über die vordere Spitze (20) der Nadelkanüle aufgebracht ist, wodurch der vorbefüllbare Behälter (12) an der Nadelkanüle (18) abgedichtet ist.

## Revendications

1. Combinaison d'un ensemble aiguille intradermique et d'un contenant préremplissable (12) ayant un réservoir (14) capable de stocker une substance pour injection dans la peau d'un animal comprenant :
une partie de raccord (16) pouvant être attachée au contenant préremplissable stockant la substance ;
une canule d'aiguille (18) supportée par ladite partie de raccord et ayant une pointe avant (20) s'étendant loin de ladite partie de raccord ;
un limiteur (22) entourant ladite canule d'aiguille et s'étendant loin de ladite partie de raccord vers ladite pointe avant de ladite canule d'aiguille,
ledit limiteur (22) comportant une surface de contact avec la peau (24) globalement plate s'étendant dans un plan globalement perpendiculaire à un axe de ladite canule d'aiguille (18) pour limiter la pénétration de la pointe avant de la canule d'aiguille dans la couche de derme de l'animal et une ouverture centrale (45) dans la surface de contact avec la peau (24) qui est axialement alignée avec la canule d'aiguille (18), ledit limiteur étant adapté pour être reçu contre la peau de l'animal afin d'administrer une injection intradermique de la substance ; **caractérisée par** ;
un manchon (36) ayant une fente (49, 53) dans celui-ci et ayant ledit contenant préremplissable disposé de manière fixe dans celui-ci avec ledit limiteur (22) faisant saillie en coulissement à partir de celui-ci,
ledit limiteur (22) étant mobile entre au moins une première position (28), où ladite surface de contact avec la peau (24) protège ladite pointe avant de ladite canule d'aiguille, et une deuxième position (30), où ladite pointe avant s'étend au-delà de ladite surface de contact avec la peau sur une distance suffisante pour administrer la substance dans la couche de derme de l'animal lors de l'enfoncement de ladite surface de contact avec la peau contre la peau de l'animal,
où ledit limiteur (22) est sollicité vers ladite première position (28) avec un moyen de type ressort (38), et
au moins un cliquet (47, 51) pouvant s'engager avec la fente (49, 53) dans ledit manchon (36) pour empêcher ledit limiteur (22) de se déplacer de ladite première position à ladite deuxième position à la suite de l'administration de l'injection intradermique,
le contenant (12) ayant un rebord (40) attaché de manière fixe au manchon (36), ledit rebord étant disposé sur une extrémité opposée audit raccord (16) et ledit moyen de type ressort (38) étant comprimé entre ledit rebord (40) et ledit limiteur (22), et
un piston (32) ayant un bouchon (35) à l'intérieur du contenant (12) et s'étendant hors du manchon (36).

2. Ensemble selon la revendication 1, dans lequel ladite pointe avant (20) de ladite canule d'aiguille (18) s'étend au-delà de ladite surface de contact avec la peau (24) sur une distance d'environ 0,5 mm à 3,0 mm lorsque ledit limiteur (22) est situé dans ladite deuxième position (30) limitant ainsi la pénétration de ladite aiguille dans la couche de derme de la peau de l'animal, de sorte que la substance soit injectée dans la couche de derme de l'animal.

3. Ensemble selon la revendication 1, dans lequel ledit manchon (36) comporte au moins une butée positionnée pour empêcher ledit limiteur (22) de coulisser au-delà de ladite deuxième position (30).

4. Ensemble selon la revendication 1, dans lequel ledit rebord (40) est attaché au manchon (36) par une pluralité de fermoirs.

5. Ensemble selon la revendication 1, comportant en outre un obturateur (44) comprenant un matériau élastique appliqué sur ladite pointe avant (20) de ladite canule d'aiguille scellant ainsi ledit contenant préremplissable (12) au niveau de ladite canule d'aiguille (18).
